# EUROPEAN PATENT APPLICATION

(11) **EP 4 640 153 A1**
(43) Date of publication of application: **29.10.2025**
(21) Application number: 24305623.1
(22) Date of filing: 23.04.2024
(51) Int. Cl.: A61B 5/352, A61B 5/363, A61B 5/00, G16H 40/63, G16H 50/20, G16H 50/30, G16H 50/70

(54) **METHOD AND SYSTEM FOR PREDICTING A RISK OF BRADYARRHYTHMIA**

(71) Applicant: Koninklijke Philips N.V., 5656 AG Eindhoven (NL)
(72) Inventor: CARBONATI, Tanner, 5656 Eindhoven (NL); FIORINA, Laurent, 5656 Eindhoven (NL); PORQUET, Pauline, 5656 Eindhoven (NL); HENRY, Christine, 5656 Eindhoven (NL)
(74) Representative: Philips Intellectual Property & Standards

(57) **Abstract**

The invention relates to a method and system for predicting a risk of bradyarrhythmia in a subject. The method comprises receiving an input cardiac signal of the subject; collecting a set of historical cardiac signal recordings for a plurality of patients; developing a machine learning model based on the set of historical cardiac signal recordings; and providing the input cardiac signal as an input to the machine-learning model to predict, based on the input cardiac signal, a bradyarrhythmia risk score indicating a risk of prior bradyarrhythmia occurrence.

## Description

### FIELD OF THE INVENTION

This invention relates to the field of bradyarrhythmia, and in particular, to a method and system for predicting a risk of bradyarrhythmia.

### BACKGROUND OF THE INVENTION

Syncope is a common condition, affecting approximately one-third of the population and accounting for 1-3% of all emergency department visits with a recurrence in 30% of cases. Initial evaluation of patients following a syncopal event remains particularly challenging as there are a wide spectrum of etiologies, ranging from benign to life-threatening. Determining the etiology of the syncope is critical for diagnosis, however, routine monitoring during initial evaluation has low diagnostic yield and requires high workup. Currently, half of all patients admitted to the hospital following a syncope are discharged without an explanation of the cause.

Bradyarrhthymias, particularly sinus node dysfunction and high-degree atrioventricular AV block, are crucial to document during evaluation of patients with unexplained syncope as they have important therapeutic and prognostic implications, such as permanent pacemaker implantation to reduce the risk of recurrent syncope. However, due to their paroxysmal nature, bradyarrhythmias may not present during initial monitoring and could warrant an electrophysiological study, which is invasive and insensitive for unmasking conduction disorders.

A non-invasive tool to accurately identify the cause of presyncope or syncope could enable appropriate monitoring strategies, such as early use of implantable loop recorders or hospitalization, to reduce the risk of recurrent syncopal events. Additionally, ruling out bradyarrhythmias as a cause of syncope may help reduce the number of unnecessary hospitalizations or non-diagnostic implantable loop recorder prescriptions.

### SUMMARY OF THE INVENTION

Therefore, it is an aim of the invention to provide a system and method for predicting a risk of bradyarrhythmia and more specifically for identifying patients who previously experienced an asystolic pause due to sinus pause or high-degree AV block.

The invention is defined by the claims.

According to an aspect of the invention, there is provided a method for predicting a risk of bradyarrhythmia in a subject.

The method comprises the steps of receiving an input cardiac signal of the subject; collecting a set of historical cardiac signal recordings for a plurality of patients; developing a machine learning model based on the set of historical cardiac signal recordings; and providing the input cardiac signal as an input to the machine-learning model to predict, based on the input cardiac signal, a bradyarrhythmia risk score indicating a risk of prior bradyarrhythmia occurrence.

In an embodiment, the historical cardiac signal recordings have a predetermined duration where no bradyarrhythmia occurs during a last portion of the predetermined duration, wherein part of these historical cardiac signals recordings presents a bradyarrhythmia event during a first portion of the predetermined duration, wherein the last portion of each historical cardiac signal recording is used as input to the deep learning model, and wherein the first portion of each historical cardiac signal recording is annotated according to whether there was a bradyarrhythmia event and is used as an output. In a particular example, the predetermined duration is between 24 hours and 30 days, the first portion of the predetermined duration is between 23 hours and 29 days and the last portion of the predetermined duration is between 1 hour and 1 day. Advantageously, the predetermined duration can be two weeks, the first portion of the predetermined duration being 13 days and the last portion of the predetermined duration being 1 day.

In another embodiment, the input cardiac signal comprises at least one of a single-lead electrocardiogram ECG signal, an ambulatory monitoring signal and a PPG signal.

Still in another embodiment, the risk score is calculated based on the following bradyarrhythmia events: (i) a daytime sinus pause ≥3 seconds, (ii) a prolonged sinus pause ≥6 seconds, (iii) a complete high-degree AV block, and (iv) a composite of any of a daytime sinus pause, a prolonged sinus pause, or a complete high-degree AV block.

The method further may comprise processing the input cardiac signal so that the input cardiac signal comprises a heart rate density plot wherein prior to providing the least one input cardiac signal as an input to the machine-learning model.

The machine-learning model may comprise a first neural network, the first neural network being trained to predict, for the heart rate density plot, a heart rate bradyarrhythmia risk prediction. It may also comprise a second neural network, the second neural network being trained to predict, for the input cardiac signal, a heart rate bradyarrhythmia risk prediction.

According to another aspect of the invention, there is provided a computer program comprising code means for implementing the method of any herein disclosed method when said program is run on a processing system.

According to another aspect of the invention, there is provided a system for predicting a risk of bradyarrhythmia in a subject.

The system comprises an input interface configured to obtain an input cardiac signal of the subject, to collect historical cardiac signal recordings for a plurality of patients; and a processing unit configured to train a machine learning model based on the historical cardiac signal recordings, and to provide the input cardiac signal as an input to the machine-learning model to predict, based on the input cardiac signal, a bradyarrhythmia risk score indicating a risk of prior bradyarrhythmia occurrence.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for predicting a risk of bradyarrhythmia in a subject according to a proposed embodiment;
Fig. 2 is a more in-depth flow diagram of a method for predicting a risk of bradyarrhythmia in a subject according to a proposed embodiment;
Fig. 3 is a simplified block diagram of a system for predicting a risk of bradyarrhythmia in a subject according to a proposed embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to predicting a risk of bradyarrhythmia in a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for predicting a risk of bradyarrhythmia in a subject. This can be achieved by utilizing a machine-learning model to process an input cardiac signal. The machine-learning model is trained to predict a bradyarrhythmia risk score indicating a risk of prior bradyarrhythmia occurrence. The risk score can then be used to determine a risk value describing a predicted risk of prior bradyarrhythmia occurrence for the subject.

Proposed concepts thus aim to determine a risk value describing a predicted risk of prior bradyarrhythmia occurrence for the subject from a mere cardiac signal of a subject, e.g. an ECG or PPG signal. This can be done via the provision of the cardiac signal to a machine-learning model trained for this purpose. The predicted risk of prior bradyarrhythmia occurrence can be understood as the risk of bradyarrhythmia occurring in the subject in the past, e.g. in the previous days.

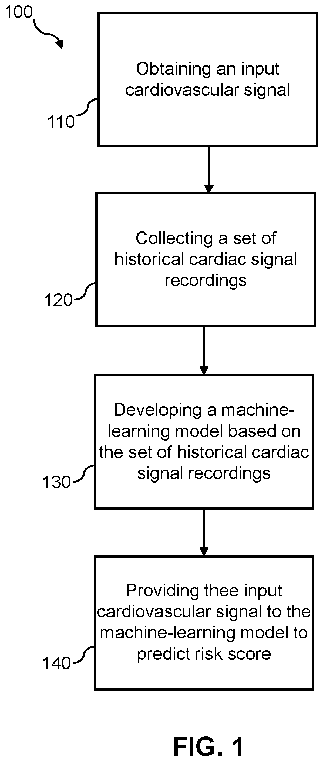

Referring now to Fig. 1, there is depicted a simplified flow diagram of a method 100 for predicting a risk of bradyarrhythmia in a subject according to a proposed embodiment.

The method 100 begins with the step 110 of obtaining an input cardiac signal of the subject. The input cardiac signal may comprise at least one of: an ECG signal; and a PPG signal. More specifically, the input cardiac signal may comprise at least one of: a single-lead ECG signal; an ambulatory monitoring signal; and a PPG signal. This allows for data from simple ECG devices, ambulatory monitors, or PPG sensors to be used. A single-lead ECG recording can be collected using many different devices and remains agnostic of biases often found in clinical data. An ambulatory monitor, for example, may comprise a Holter monitor or Holter patch. An ambulatory monitoring signal may comprise at least one of: an ECG signal; and a PPG signal. In this embodiment, the input cardiac signal comprises cardiovascular data for a window of a predetermined number of hours.

The method then comprises a step 120 of collecting a set of historical cardiac signal recordings having a predetermined duration for a plurality of patients, with no bradyarrhythmia during a last portion of the predetermined duration, part of these extended cardiac signals recordings presenting a bradyarrhythmia during a first portion of the predetermined duration. The method also comprises a step of developing a deep learning model using the last portion of the predetermined duration of the set of extended cardiac signal recordings knowing if and when a bradyarrhythmia has been detected for each extended cardiac signal recording of the set so as to predict the risk of bradyarrhythmia in the first portion of the predetermined duration. The predetermined duration is preferably between 24 hours and 30 days, the first portion of the predetermined duration being between 23 hours and 29 days and the last portion of the predetermined duration being between 1 hour and 1 day. For example, two-week cardiac signals recordings are used to derive the deep learning model inputs and outputs. The last 24 hours of each recording (which have no asystolic pause) are used as input to a deep learning model. Each recording is annotated according to whether there was any asystolic event documented in the previous 13 days and used it as an output.

In step 130, a machine learning model is developed based on the set of historical cardiac signal recordings. The machine learning model is a deep learning-based model that can be deployed in the cloud or embedded into a device and that has been developed and internally and externally validated to identify patients who previously experienced an asystolic pause due to either a daytime sinus pause ≥3 s, anytime sinus pause ≥6 s, or high-degree AV block from a single-lead ambulatory ECG. Two-week ambulatory ECG recordings were used to derive the model inputs and outputs. The last 24 hours of each recording (which had no asystolic pause) were used as input to a deep learning model. We then annotated each recording according to whether there was any asystolic event documented in the previous 13 days and used it as an output.

The model was developed and internally validated using an internal dataset consisting of ambulatory ECG recordings uploaded to the Cardiologs platform by multiple Independent Diagnostic Testing Facilities (IDTFs) from the US between 1 January 2019 and 1 September 2023. The internal dataset was divided into a development and held-out validation set in the following way: all recordings collected before 1 April 2023 were used for model development and all collected thereafter were held-out for internal validation. The development dataset was randomly split into a training set (80%) and tuning set (20%). The tuning set was used to select hyperparameters and operating points from the training process.

To assess the generalizability of the model across different IDTFs with different patient populations, data collection strategies, and devices we validated the model using a fully independent external validation dataset. The external validation set consisted of recordings collected from a separate IDTF between 1 January 2019 and 1 September 2023. All recordings in the external validation set were excluded from model development. Any recordings with an asystolic pause during the last 24 hours were excluded from the validation sets. Ambulatory recordings were collected from numerous manufacturers, which consisted of single and multi-lead classic Holter and patch-based monitors. Given the variability of electrode positioning across patch-based and multi-lead Holters we did not select a specific lead derivation, but rather a random channel from each recording was selected during training and the first available channel from the device was used during validation. All ECG recordings were resampled to 250 Hz.

In step 140, the input cardiac signal is provided as an input to the machine-learning model, the machine-learning model being trained to predict, based on the input cardiac signal, a bradyarrhythmia risk score indicating a risk of prior bradyarrhythmia occurrence. A risk value describing a predicted risk of prior bradyarrhythmia occurrence for the subject can be determined based on the bradyarrhythmia score. A predicted risk of prior bradyarrhythmia occurrence may be understood as the risk of bradyarrhythmia occurring in the subject in a next predetermined time frame. The bradyarrhythmia risk score can simply taken as the risk value such as a percentage-likelihood of arrythmia occurrence in the past. The risk value may be an adjusted risk score, taking into account one or more further parameters, e.g. the risk score may be multiplied or divided by a certain adjustment value based on the subject's recent medical history, clinical diagnosis, or demographic data.

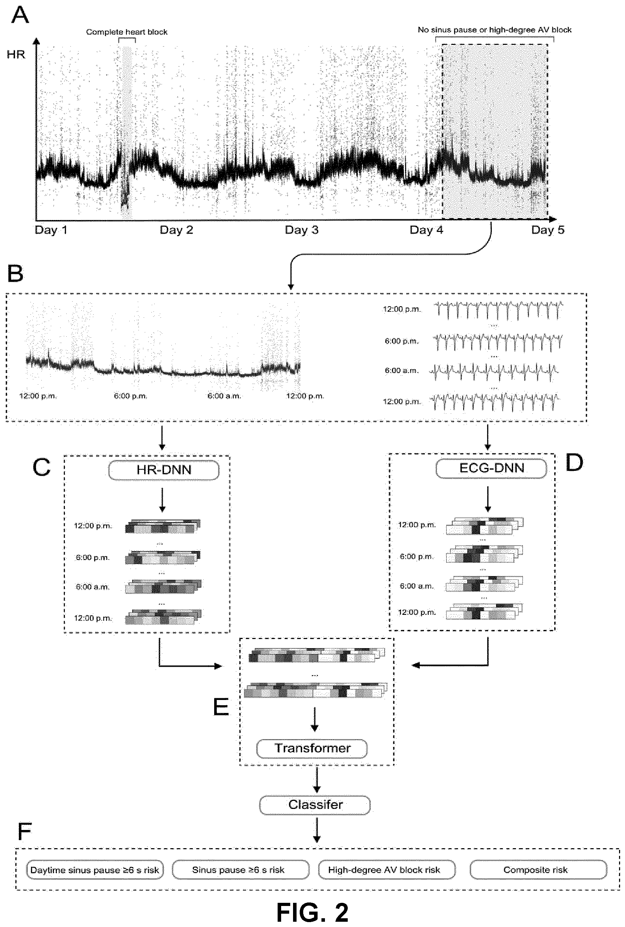

Referring now to Fig. 2, there is depicted a more in-depth flow diagram of a method 200 for predicting a risk of bradyarrhythmia in a subject and more specifically to the deep learning-based model. In this embodiment, the cardiac signal is an ECG waveform in the form of a heart rate HR signal having a duration of 5 days.

In a first stage, the deep learning-based model is an ensemble of two algorithms, both utilizing a single-lead ambulatory ECG. The two algorithms include: a first deep neural network DNN, which uses as input a heart rate density plot and a second DNN, which uses ECG waveform data as input

A heart rate density plot HRDP (B left part) having a duration of 24 hours is derived from the 5^{th} day of the HR signal (A). A heart rate density plot is a three-dimensional representation of the instantaneous heart rate during the monitoring period. The x-axis represents time, the y-axis represents HR and the z-axis consists of three channels corresponding to each beat's classification as either: sinus, premature ventricular contraction, or premature atrial contraction. A first DNN is used, which consists of a 2-dimensional convolutional neural network CNN encoder, a recurrent neural network, an attention-based pooling layer, and a classifier. The CNN is based on the DenseNet-121 architecture, which takes as input the HRDP to extract spatial feature maps from the recording. An attention pooling layer is used to aggregate spatial feature maps across the HR-axis (y-axis) resulting in temporal feature maps (C). A long short-term cell, which is a specific type of recurrent neural network (typically for temporal/sequential information), is used to exploit relationships across the temporal HR features. A final attention pooling layer is then used to generate a global feature representation of the HRDP. The classifier is composed of a dropout layer, fully connected layer, and sigmoid activation to predict an HR score.

A second DNN is applied directly to the ECG waveform data from the recording (B right part). The second DNN comprises an ECG-strip encoder, a strip-pooling layer, a transformer encoder and a classifier. The ECG-strip encoder is a 1-dimensional CNN based on the SENet architecture, which takes as input a 10-second ECG waveform strip to generate a single strip-embedding (D). During training, a longer 30-minute segment is randomly extracted from the ambulatory ECG recording and partitioned into a stack of 180 10-second strips. Each strip is passed to the ECG-strip encoder to generate 180 strip embeddings. A transformer encoder, composed of two multi-headed attention layers, is then used to learn interactions between the sequence of strip embeddings. A global pooling layer is applied to the output of the transformer to generate a global feature representation of the 30-minute ECG segment. The classifier is composed of a dropout layer, fully connected layer, and sigmoid activation to generate an ECG-segment score from the global feature representation. During inference, each recording is partitioned into non-overlapping 30-minute strips.

In a second stage, a joint feature representation is learned by combining the intermediate features extracted from the ECG-strip encoder and first DNN encoder (C & D). The intermediate features extracted from the ECG-strip encoder and the first DNN encoder are concatenated as a temporal feature vector which is used as input to a transformer module (E). The transformer module is composed of four multi-headed attention layers to learn interactions between the ECG and HR derived features and generate a joint feature representation. The joint feature representation is then used as input to a fusion classifier model (F). The fusion classifier model is a third DNN consisting of a layer normalization layer and four fully connected layers with dropout in between each layer. The last fully connected layer outputs a risk score for each endpoint. Note that the extracted features from the first stage are used as intermediate inputs in the second stage, and the weights from the first stage are used as the initial weights to initialize the third DNN architecture in the second stage.

Four risk scores are generated for the following events to occur during the initial 4 days of monitoring:
a daytime sinus pause ≥3 seconds,
anytime prolonged sinus pause ≥6 seconds,
a high degree atrioventricular block, and
a composite of any of daytime sinus pause, prolonged sinus pause, or high degree atrioventricular block.

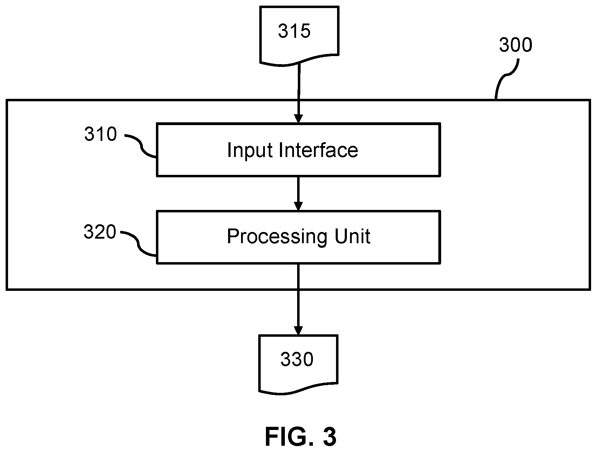

Referring now to Fig. 3, there is depicted a system 300 for predicting a risk of bradyarrhythmia in a subject according to a proposed embodiment. The system 300 comprises an input interface 310 and a processor 320.

The system 300 is configured to predict a risk of bradyarrhythmia in a subject by processing inputs 315. The inputs 315 comprise an input cardiac signal of the subject. The input interface 310 obtains the inputs 315 and the processor 320 is then configured to: provide the input cardiac signal as an input to a machine-learning model, the machine learning model being trained to predict, based on the input cardiac signal, a bradyarrhythmia risk score indicating a risk of prior bradyarrhythmia occurrence; and based on this risk score, determine an output 330. The output 330 comprises a risk value describing a predicted risk of prior bradyarrhythmia occurrence for the subject.

The methods of Figs. 1-2, and the system of Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for predicting a risk of bradyarrhythmia in a subject, the method comprising:
receiving an input cardiac signal of the subject;
collecting a set of historical cardiac signal recordings for a plurality of patients;
developing a machine learning model based on the set of historical cardiac signal recordings; and
providing the input cardiac signal as an input to the machine-learning model to predict, based on the input cardiac signal, a bradyarrhythmia risk score indicating a risk of prior bradyarrhythmia occurrence.

2. The method of claim 1, wherein the historical cardiac signal recordings have a predetermined duration where no bradyarrhythmia occurs during a last portion of the predetermined duration, wherein part of these historical cardiac signals recordings presents a bradyarrhythmia event during a first portion of the predetermined duration, wherein the last portion of each historical cardiac signal recording is used as input to the deep learning model, and wherein the first portion of each historical cardiac signal recording is annotated according to whether there was a bradyarrhythmia event and is used as an output.

3. The method of claim 1, wherein the predetermined duration is between 24 hours and 30 days, the first portion of the predetermined duration is between 23 hours and 29 days and the last portion of the predetermined duration is between 1 hour and 1 day.

4. The method of claim 3, wherein the predetermined duration is two weeks, the first portion of the predetermined duration is 13 days and the last portion of the predetermined duration is 1 day.

5. The method of any prior claim, wherein the input cardiac signal comprises at least one of a single-lead ECG signal, an ambulatory monitoring signal and a PPG signal.

6. The method of any prior claim, wherein the risk score is calculated based on the following bradyarrhythmia events: (i) a daytime sinus pause ≥3 seconds, (ii) a prolonged sinus pause ≥6 seconds, (iii) a high-degree atrioventricular block, and (iv) a composite of any of a daytime sinus pause, a prolonged sinus pause, or a complete high-degree atrioventricular block.

7. The method of any prior claim, wherein prior to providing the least one input cardiac signal as an input to the machine-learning model, the method further comprises processing the input cardiac signal so that the input cardiac signal comprises a heart rate density plot.

8. The method of any prior claim, wherein the machine-learning model comprises a first neural network, the first neural network being trained to predict, for the heart rate density plot, a heart rate bradyarrhythmia risk prediction.

9. The method of any prior claim, wherein the machine-learning model comprises a second neural network, the second neural network being trained to predict, for the input cardiac signal, a heart rate bradyarrhythmia risk prediction.

10. A computer program comprising code means for implementing the method of any preceding claim when said program is run on a processing system.

11. A system for predicting a risk of bradyarrhythmia in a subject, the system comprising:
an input interface configured to:
obtain an input cardiac signal of the subject;
collect historical cardiac signal recordings for a plurality of patients; and
a processing unit configured to:
train a machine learning model based on the historical cardiac signal recordings; and
provide the input cardiac signal as an input to the machine-learning model to predict, based on the input cardiac signal, a bradyarrhythmia risk score indicating a risk of prior bradyarrhythmia occurrence.

12. A monitoring system comprising a cardiovascular monitor and the system of claim 11.
